(19) Europäisches Patentamt
European Patent Office
Office européen des brevets

(11) Numéro de publication : **0 433 189 B1**

# (12) FASCICULE DE BREVET EUROPEEN

(45) Date de publication du fascicule du brevet : **23.06.93 Bulletin 93/25**

(51) Int. Cl.$^5$ : **C07C 67/38,** C07C 69/593

(21) Numéro de dépôt : **90420536.6**

(22) Date de dépôt : **11.12.90**

## (54) Procédé de préparation de diesters de l'acide hexènedioique.

(30) Priorité : **13.12.89 FR 8916753**

(43) Date de publication de la demande : **19.06.91 Bulletin 91/25**

(45) Mention de la délivrance du brevet : **23.06.93 Bulletin 93/25**

(84) Etats contractants désignés : **BE DE ES FR GB IT NL**

(56) Documents cités :
**EP-A- 0 272 191**
**US-A- 3 367 961**
**US-A- 4 060 547**
**US-A- 4 633 015**

(73) Titulaire : **RHONE-POULENC CHIMIE**
**25, quai Paul Doumer**
**F-92408 Courbevoie Cédex (FR)**

(72) Inventeur : **Denis, Philippe**
**16, Allée des Troenes**
**F-69150 Decines (FR)**

(74) Mandataire : **Vignally, Noel et al**
**Rhône-Poulenc Chimie Direction de la Propriété Industrielle Centre de Recherches des Carrières B.P. 62**
**F-69192 Saint-Fons Cédex (FR)**

EP 0 433 189 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet un procédé de préparation de diesters de l'acide hexènedioïque-1,6. Ces diesters peuvent être hydrogénés en diesters de l'acide adipique correspondants ou adipates qui peuvent alors être hydrolysés pour former l'acide adipique. L'acide adipique, l'une des matières premières du nylon 66 est produit avec de forts tonnages et de ce seul fait, toute nouvelle voie d'accès à ce diacide et/ou à ses dérivés présente un intérêt de principe immédiatement perceptible.

La présente invention a plus spécifiquement pour objet un procédé de préparation de diesters de l'acide hexène-3 dioïque-1,6 par réaction du monoxyde de carbone et d'un alcool sur au moins un butène disubstitué par des groupes acyloxy en présence d'un catalyseur à base de palladium.

La préparation d'un monoester d'un alcool et d'un acide buténoïque par réaction du monoxyde de carbone et d'un alcool sur un monoester d'un alcool allylique en présence d'un catalyseur à base de palladium est décrite dans le brevet américain n° 3,367,961. Ainsi il est possible d'obtenir du vinylacétate d'éthyle à partir d'acétate d'allyle, de monoxyde de carbone et d'éthanol en présence de chlorure de palladium et de palladium déposé sur charbon.

Il est également indiqué dans le brevet américain n° 4,611,082 que la carbonylation d'une solution du diacétoxy-1,4 butène-2 dans un solvant aprotique, polaire et non basique choisi dans le groupe constitué par les nitriles, le bis(méthoxy-2) butène-2, le bis(méthoxy-2 éthyl)éther et le chlorure de méthylène à 80-140°C en présence d'un halogénure d'un métal de transition n'est pratiquement pas observée et qu'en présence d'un alcool les vitesses augmentent et sont comparables à celles constatées pour la carbonylation du butène-2 diol-1,4. A propos de ce dernier substrat mentionné il est également indiqué qu'il ne permet pas d'atteindre dans les conditions précitées des rendements satisfaisants en produits linéaires de carbonylation et, dans ce contexte, préférence est donnée aux substrats substitués en position 1,4 par des groupements alcoxy.

Or le diacétoxy-1,4 butène-2 est aisément accessible par acétoxylation du butadiène. Il serait donc hautement souhaitable de pouvoir disposer d'un procédé permettant d'obtenir avec une efficacité élevée les diesters de l'acide hexène-3 dioïque-1,6 à partir du diacétoxy-1,4 butène-2, par exemple, et plus généralement de butènes disubstitués par des groupes acyloxy.

Dans la demande européenne n° 89/4201995 il est proposé un procédé de préparation de diesters de l'acide hexène-3 dioïque par réaction du monoxyde de carbone et d'un alcool sur au moins un butène disubstitué par des groupes acyloxy en présence d'un catalyseur à base de palladium caractérisé en ce que la réaction est conduite également en présence d'un halogénure d'onium quaternaire d'un élément du groupe VB choisi parmi l'azote et le phosphore, ledit élément étant tétracoordonné à des atomes de carbone, l'azote pouvant être coordonné à deux atomes de phosphore pentavalents, l'anion halogénure étant choisi parmi le chlorure et le bromure.

Le procédé en cause qui conduit à des résultats appréciables tant sur le plan de l'activité que sur celui de la sélectivité en produit dicarbonylé linéaire présente cependant l'inconvénient de requérir la présence d'au moins un halogénure d'onium quaternaire au sens indiqué ci-avant, promoteurs relativement onéreux ou peu disponibles et susceptibles de se dégrader lors un usage prolongé.

C'est pourquoi il peut s'avérer utile de proposer une alternative audit procédé par laquelle le promoteur organique halogéné n'est plus indispensable et tout ou partie dudit promoteur peut être remplacée par un promoteur halogéné minéral, plus disponible et relativement plus stable lors d'un usage prolongé.

La présente invention a donc pour objet un procédé de préparation de diesters de l'acide hexène-3 dioïque par réaction du monoxyde de carbone et d'un alcool sur au moins un butène disubstitué par des groupes acyloxy en présence d'un catalyseur à base de palladium et d'un halogénure caractérisé en ce que la réaction est conduite dans un solvant polaire aprotique basique et en présence d'au moins un halogénure minéral dont le cation est choisi parmi les cations des métaux alcalins et les cations des métaux alcalino-terreux, l'anion halogénure étant choisi parmi le chlorure et le bromure.

Il a en effet été trouvé de manière tout à fait surprenante qu'un tel procédé permet de réaliser la dicarbonylation dans des conditions de pression et de température acceptables à l'échelle industrielle, avec une sélectivité appréciable en produit dicarbonylé linéaire, les proportions de produit monocarbonylé et de produits dicarbonylés branchés étant minimes.

Le procédé en cause peut être représenté par le schéma réactionnel suivant, lorsqu'on part d'un butène-2 disubstitué en 1,4 par des groupes acyloxy,

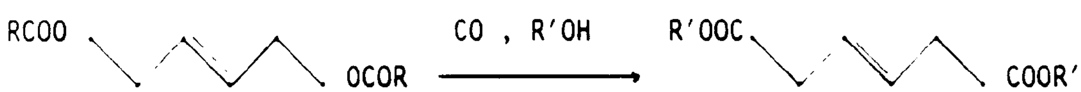

dans lequel :

- R représente :
  . un radical alkyle linéaire ou ramifié, contenant de 1 à 12 atomes de carbone, éventuellement substitué par un groupe phényle ;
  ou
  . un radical aryle contenant de 6 à 10 atomes de carbone, éventuellement substitué par un ou deux radicaux alkyles contenant de 1 à 4 atomes de carbone ;

et peut comporter de 1 à 3 substituants choisis parmi les atomes de fluor et de chlore et les groupements dialkylamino et N,N-dialkylamido dont les radicaux alkyles renferment au plus 4 atomes de carbone.

- R', identique à ou différent de R, représente :
  . un radical alkyle linéaire ou ramifié contenant de 1 à 12 atomes de carbone, éventuellement substitué par un groupe phényle ;
  ou
  . un radical aryle contenant de 6 à 10 atomes de carbone, éventuellement substitué par un ou deux radicaux alkyles contenant de 1 à 4 atomes de carbone ;

et peut comporter de 1 à 3 substituants choisis parmi les atomes de fluor, de brome et de chlore.

Le procédé, selon la présente invention, requiert la mise en oeuvre d'au moins un butène disubstitué par des groupes acyloxy. Par groupe acyloxy, on entend des groupes de formule RCOO- dans laquelle R a la signification donnée précédemment ; par butènes disubstitués on entend les composés du butène-2 substitués en position 1 et 4 et les composés du butène-1 substitués en positions 3 et 4. Bien entendu des mélanges de butène-2 disubstitués par des groupes acyloxy de nature distincte, des mélanges de butène-1 disubstitués par des groupes acyloxy de nature distincte ou des mélanges de butène-2 et de butène-1 disubstitués peuvent être mis en oeuvre dans le cadre du présent procédé.

Il a en effet été constaté par la Demanderesse que la sélectivité en diester linéaire est sensiblement la même que l'on parte d'un butène-2 disubstitué par des groupes acyloxy en position 1,4 ou d'un butène-1 disubstitué par des groupes acyloxy en positions 3 et 4.

A titre d'exemples de butènes disubstitués par des groupes acyloxy on peut citer : les diacétoxybutènes, les dipropionyloxybutènes, les dibutyryloxybutènes et les dibenzoyloxybutènes.

Le diacétoxy-1,4 butène-2, le diacétoxy-3,4 butène-1 et leurs mélanges conviennent plus particulièrement bien à la mise en oeuvre de la présente invention.

Le procédé selon la présente invention requiert également la mise en oeuvre d'un alcool de formule R'OH dans laquelle R' a la signification donnée précédemment.

A titre d'exemple d'alcool convenant à la mise en oeuvre du procédé on peut citer :
le méthanol, l'éthanol, l'isopropanol, le n-propanol, le tertiobutanol, le n-hexanol, l'éthyl-2 hexanol, le nonanol-1, le dodécanol, le phényléthanol, le phénol et le trifluoroéthanol.

On utilise avantageusement un alcanol dont le radical R' comporte au maximum 12 atomes de carbone et, de préférence, 4 atomes de carbone.

La quantité d'alcanol à mettre en oeuvre dans le cadre du présent procédé n'est pas critique et peut varier dans de larges limites.

Pour une bonne mise en oeuvre de la réaction le rapport molaire de l'alcool au butène disubstitué sera compris entre 1 et 100 et, de préférence entre 1 et 50.

Le procédé selon la présente invention est conduit en présence d'un catalyseur à base de palladium.

Bien que la nature précise de l'espèce (ou des espèces) catalytiquement active(s) dans la réaction en cause ne soit pas totalement élucidée, la Demanderesse a constaté que divers composés du palladium et le palladium métallique sont susceptibles d'être des précurseurs utiles dans la mise en oeuvre du présent procédé.

Parmi les sources de palladium pouvant être mises en oeuvre pour réaliser le procédé faisant l'objet de l'invention, on peut citer :

. le palladium métallique déposé le cas échéant sur un support, tel que le charbon, l'alumine, ou la silice,
. $PdCl_2$, $Pd(OAc)_2$
. les sels ou les complexes $\pi$-allyliques de palladium, dont l'anion coordonné au cation Pd est choisi parmi les anions suivants : carboxylates tels que formiate, acétate, propionate, benzoate ; acétylacétonate, halogénures tels que $Cl^-$ et $Br^-$ et de préférence $Cl^-$ ;

On recourt avantageusement au chlorure de palladium.

La quantité précise de catalyseur à mettre en oeuvre, qui peut varier dans de larges limites, dépendra avant tout d'un compromis entre l'efficacité souhaitée et la dépense en catalyseur et des autres conditions choisies pour la réaction. En général de bons résultats peuvent être obtenus avec une concentration en palladium dans le milieu réactionnel, comprise entre $10^{-3}$ et une mol/l. De préférence, cette concentration est comprise entre $2.10^{-3}$ et $5.10^{-2}$ mol/l.

L'une des caractéristiques essentielles du présent procédé réside dans le fait que la réaction est conduite dans un solvant polaire, aprotique et basique.

Par solvant polaire, aprotique et basique la Demanderesse entend les composés de formule (I) :

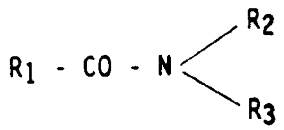

(I)

dans laquelle $R_1$, $R_2$ et $R_3$, identiques ou différents représentent un radical alkyle, un radical cycloalkyle, un radical aralkyle ou un radical aryle monocyclique ayant au plus 10 atomes de carbone, deux radicaux $R_1$, $R_2$ ou $R_3$, pouvant constituer ensemble un radical unique divalent - $(CH_2)y$ -, y étant un entier compris entre 3 et 12, $R_1$ pouvant en outre représenter un radical

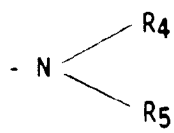

(II)

dans lequel $R_4$ et $R_5$, identiques ou différents représentent un radical alkyle ayant au maximum 4 atomes de carbone.

A titre d'exemple de tels solvants, on peut citer :

la tétraméthylurée, le N,N-diméthylacétamide, le N,N-diéthylacétamide, le N,N-dicyclohexylacétamide, le N,N-diméthylpropionamide, le N-N-diéthylpropionamide, le N,N-diéthyl-n-butyramide, le N,N-diméthylbenzamide, le N,N-dicyclohexylbenzamide, le N,N-diéthyl-m-toluamide, la N-acétylpyrrolidine, la n-acétylpipéridine, la N-(n-butyryl)pipéridine, la N-méthylpyrrolidone-2, la N-éthylpyrrolidone-2, la N-méthylpipéridone-2, le N-méthyl-epsiloncaprolactame.

La N-méthylpyrrolidone-2 convient particulièrement bien à la mise en oeuvre du présent procédé.

En général la quantité de solvant représente au moins 10% en volume du milieu réactionnel ; de bons résultats sont obtenus lorsqu'on en utilise de l'ordre de 20% à 85% en volume.

Selon une autre caractéristique du présent procédé, la réaction est conduite également en présence d'un halogénure dont le cation est choisi parmi les cations des métaux alcalins et les cations des métaux alcalino-terreux, l'anion halogénure étant choisi parmi le chlorure et le bromure.

A titre d'exemples de tels halogénures on peut citer : LiCl, LiBr et $CaCl_2$.

Il a été constaté que l'effet bénéfique apporté par la présence dans le milieu de carbonylation d'un chlorure ou bromure alcalin ou alcalino-terreux est sensible à partir d'un rapport molaire $Cl^-$ (ou $Br^-$)/ palladium de 0,5 ; notamment un effet particulièrement intéressant a été constaté lorsque ledit rapport est compris entre 1 et 50, un rapport plus élevé, n'étant toutefois pas nocif pour la réaction.

Bien entendu, on peut mettre en oeuvre dans le cadre de la présente invention un mélange de tels halogénures minéraux ou un mélange d'au moins un halogénure d'onium quaternaire au sens rappelé en tête du présent mémoire.

La réaction pourra être généralement conduite en phase liquide à une température comprise entre 50 et 150°C, de préférence entre 80 et 130°C, sous une pression d'oxyde de carbone comprise entre 20 et 250 bar, de préférence entre 90 et 180 bar.

Des gaz inertes, tels que l'azote, l'argon ou le gaz carbonique, peuvent être présents à côté de l'oxyde de carbone.

En fin de réaction ou du temps imparti à la réaction on récupère le diester recherché par tout moyen approprié, par exemple par extraction et/ou distillation.

Les exemples ci-après illustrent l'invention ; les rendements sont exprimés par rapport au diacétoxy butène chargé.

EXEMPLE 1 à 5 ; Essais témoins (a) à (d) :

Dans un autoclave en acier inoxydable (Hastelloy B2) de 125 cm3, préalablement purgé à l'argon, on introduit :

. 4,3 g (25 mmol) de diacétoxy-1,4 butène-2

. du méthanol
. 1 mat-g de palladium sous forme de $PdCl_2$
. le cas échéant 17 mmol de chlorure de calcium (de chlorure de lithium ou de bromure de lithium)
. de la N-méthylpyrrolidone-2 (NMP) ou, le cas échéant, un autre solvant.

L'autoclave est fermé hermétiquement, placé dans un four agité et raccordé à l'alimentation de gaz sous pression. On purge le réacteur à froid avec de l'oxyde de carbone et on le porte à 100°C. On régule ensuite la pression à 120 bar. Lorsque l'absorption de l'oxyde de carbone est terminée, l'autoclave est refroidi et dégazé.

Le mélange réactionnel est alors estérifié par du méthanol puis analysé par chromatographie en phase gazeuse.

Les conditions particulières ainsi que les résultats obtenus figurent au tableau ci-après dans lequel t(mn) représente la durée de l'absorption et HD (%) représente la quantité molaire d'hexène-3 dioate de méthyle formée pour 100 moles de diacétoxy-1,4 butène chargées.

Dans l'ensemble des essais le taux de transformation du diacétoxy-1,4 butène est de l'ordre de 100%.

TABLEAU

| Réf. | Nature de l'halogénure | MeOH | NMP | t(mn) | HD(%) |
|---|---|---|---|---|---|
| 1 | néant | 53 mmol | 20 cm3 | 390 | 53,5 |
| a(*) | Li I | 53 mmol | 20 cm3 | 180 | 0 |
| 2 | Li Br | 53 mmol | 20 cm3 | 120 | 76 |
| 3 | Li Cl | 53 mmol | 20 cm3 | 90 | 95 |
| 4 | Ca $Cl_2$ | 53 mmol | 20 cm3 | 60 | 95 |
| 5 | Ca $Cl_2$ | 10 cm3 | 10 cm3 | 50 | 72,5 |
| b | Ca $Cl_2$ | 20 cm3 | 0 | 120 | 0 |
| c(*) | Ca $Cl_2$ | 0 | 20 cm3 | 120 | 0 |
| d | Li Cl | 10 cm3 | toluène 10 cm3 | 60 | 26 |

(*) dans ces essais on détecte la présence de l'ordre de 30 à 40 % de pentadiénoate de méthyle.

## Revendications

1. Procédé de préparation de diesters de l'acide hexène-3 dioïque par réaction du monoxyde de carbone et d'un alcool sur au moins un butène disubstitué par des groupes alcyloxy en présence d'un catalyseur à base de palladium et d'halogènure caractérisé en ce que la réaction est conduite dans un solvant polaire aprotique basique et en présence d'au moins un halogénure minéral dont le cation est choisi parmi les cations des métaux alcalins et les cations des métaux alcalino-terreux, l'anion halogénure étant choisi parmi le chlorure et le bromure.

2. Procédé selon la revendication 1, caractérisé en ce que le solvant est choisi parmi les composés de formule (I) :

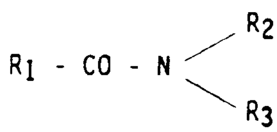

(I)

dans laquelle $R_1$, $R_2$ et $R_3$, identiques ou différents représentent un radical alkyle, un radical cycloalkyle, un radical aralkyle ou un radical aryle monocyclique ayant au plus 10 atomes de carbone, deux radicaux $R_1$, $R_2$ ou $R_3$, pouvant constituer ensemble un radical unique divalent - $(CH_2)y$ -, y étant un entier compris entre 3 et 12, $R_1$ pouvant en outre représenter un radical

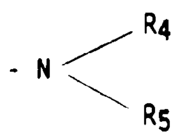

(II)

dans lequel $R_4$ et $R_5$, identiques ou différents représentent un radical alkyle ayant au maximum 4 atomes de carbone.

3. Procédé selon la revendication 1 ou 2 caractérisé en ce que la quantité de solvant représente au moins 10% en volume du milieu réactionnel.

4. Procédé selon l'une quelconque des revendications précédentes caractérisé en ce que le solvant est la N-méthylpyrrolidone-2.

5. Procédé selon l'une quelconque des revendications précédentes, caractérisé en ce que le rapport molaire de l'anion chlorure (ou bromure) au palladium est compris entre 1 et 50.

6. Procédé selon l'une quelconque des revendications précédentes, caractérisé en ce que la concentration en palladium dans le milieu réactionnel est comprise entre $10^{-3}$ et 1 mol/l.

7. Procédé selon l'une quelconque des revendications précédentes, caractérisé en ce que le rapport molaire de l'alcool au butène disubstitué est compris entre 1 et 100 et, de préférence, entre 1 et 50.

8. Procédé selon l'une quelconque des revendications précédentes, caractérisé en ce que la température de réaction est comprise entre 50 et 150°C et, de préférence, entre 80 et 130°C.

9. Procédé selon l'une quelconque des revendications précédentes, caractérisé en ce que la pression est comprise entre 20 et 250 bar et, de préférence, entre 90 et 180 bar.

10. Procédé selon l'une quelconque des revendications précédentes, caractérisé en ce que le butène disubstitué est choisi parmi le diacétoxy-1,4 butène-2 et le diacétoxy-3,4 butène-1 et leurs mélanges.

11. Procédé selon l'une quelconque des revendications précédentes, caractérisé en ce que le palladium est engagé sous forme de chlorure de palladium.

**Patentansprüche**

1. Verfahren zur Herstellung von Diestern der Hex-3-en-disäure durch Umsetzung von Kohlenmonoxyd und einem Alkohol mit zumindest einem durch Acyloxygruppen disubstituierten Buten in Gegenwart eines Katalysators auf Basis von Palladium und Halogenid, dadurch gekennzeichnet, daß die Umsetzung in einem basischen aprotischen polaren Lösungsmittel und in Gegenwart von zumindest einem mineralischen Halogenid durchgeführt wird, dessen Kation unter den Kationen der Alkalimetalle und den Kationen der Erdalkalimetalle ausgewählt wird, wobei das Halogenidanion unter dem Chlorid und dem Bromid ausgewählt wird.

2. Verfahren gemäß Anspruch 1, dadurch gekennzeichnet, daß das Lösungsmittel unter den Verbindungen der Formel (I):

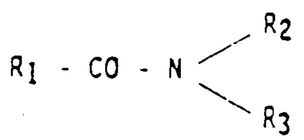

(I)

ausgewählt wird, worin $R_1$, $R_2$ und $R_3$, identisch oder voneinander verschieden, einen Alkylrest, einen Cycloalkylrest, einen Aralkylrest oder einen monocyclischen Arylrest mit höchstens 10 Kohlenstoffatomen bedeuten, wobei zwei Reste $R_1$, $R_2$ oder $R_3$ gemeinsam einen einzigen zweiwertigen Rest - $(CH_2)_y$ - bilden können, worin y eine ganze Zahl zwischen 3 und 12 ist, und wobei $R_1$ außerdem einen Rest

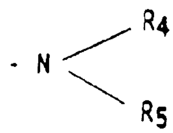

(II)

darstellen kann, worin $R_4$ und $R_5$, identisch oder verschieden, einen Alkylrest mit höchstens 4 Kohlenstoffatomen bedeuten.

3. Verfahren gemäß Anspruch 1 oder 2, dadurch gekennzeichnet, daß die Menge des Lösungsmittels zumindest 10 Vol.% des Reaktionsmilieus beträgt.

4. Verfahren gemäß einem der vorhergehenden Ansprüche, dadurch gekennzeichnet, daß das Lösungsmittel das N-Methyl-2-pyrrolidon ist.

5. Verfahren gemäß einem der vorhergehenden Ansprüche, dadurch gekennzeichnet, daß das Mol-Verhältnis des Chlorid-(oder Bromid)anions zu dem Palladium zwischen 1 und 50 beträgt.

6. Verfahren gemäß einem der vorhergehenden Ansprüche, dadurch gekennzeichnet, daß die Konzentration an Palladium in dem Reaktionsmilieu zwischen $10^{-3}$ und 1 Mol pro Liter beträgt.

7. Verfahren gemäß einem der vorhergehenden Ansprüche, dadurch gekennzeichnet, daß das Mol-Verhältnis des Alkohols zu dem disubstituierten Buten zwischen 1 und 100, vorzugsweise zwischen 1 und 50, beträgt.

8. Verfahren gemäß einem der vorhergehenden Ansprüche, dadurch gekennzeichnet, daß die Reaktionstemperatur zwischen 50 und 150°C und vorzugsweise zwischen 80 und 130°C beträgt.

9. Verfahren gemäß einem der vorhergehenden Ansprüche, dadurch gekennzeichnet, daß der Druck zwischen 20 und 250 bar und vorzugsweise zwischen 90 und 180 bar liegt.

10. Verfahren gemäß einem der vorhergehenden Ansprüche, dadurch gekennzeichnet, daß das disubstituierte Buten unter 1,4-Diacetoxybut-2-en und 3,4-Diacetoxybut-1-en und deren Gemischen ausgewählt wird.

**11.** Verfahren gemäß einem der vorhergehenden Ansprüche, dadurch gekennzeichnet, daß das Palladium in Form von Palladiumchlorid eingesetzt wird.

## Claims

**1.** Process for the preparation of diesters of 3-hexenedioic acid by reaction of carbon monoxide and of an alcohol with at least one butene disubstituted by acyloxy groups, in the presence of a catalyst based on palladium and halide, characterised in that the reaction is conducted in a basic aprotic polar solvent and in the presence of at least one inorganic halide whose cation is chosen from alkali metal cations and the cations of alkaline-earth metals, the halide anion being chosen from chloride and bromide.

**2.** Process according to Claim 1, characterised in that the solvent is chosen from the compounds of formula (I):

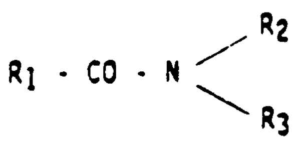

(I)

in which $R_1$, $R_2$ and $R_3$, which are identical or different, denote an alkyl radical, a cycloalkyl radical, an aralkyl radical or a monocyclic aryl radical containing not more than 10 carbon atoms, it being possible for two radicals $R_1$, $R_2$ or $R_3$ together to form a single divalent radical $-(CH_2)_y-$, y being an integer between 3 and 12, it being additionally possible for $R_1$ to denote a radical

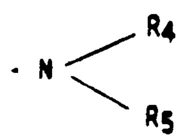

(II)

in which $R_4$ and $R_5$, which are identical or different, denote an alkyl radical containing not more than 4 carbon atoms.

**3.** Process according to Claim 1 or 2, characterised in that the quantity of solvent represents at least 10 % by volume of the reaction mixture.

**4.** Process according to any one of the preceding claims, characterised in that the solvent is N-methyl-2-pyrrolidone.

**5.** Process according to any one of the preceding claims, characterised in that the molar ratio of the chloride (or bromide) anion to palladium is between 1 and 50.

**6.** Process according to any one of the preceding claims, characterised in that the palladium concentration in the reaction mixture is between $10^{-3}$ and 1 mol/l.

**7.** Process according to any one of the preceding claims, characterised in that the molar ratio of the alcohol to the disubstituted butene is between 1 and 100 and, preferably, between 1 and 50.

**8.** Process according to any one of the preceding claims, characterised in that the reaction temperature is between 50 and 150°C and, preferably, between 80 and 130°C.

**9.** Process according to any one of the preceding claims, characterised in that the pressure is between 20 and 250 bar and, preferably, between 90 and 180 bar.

**10.** Process according to any one of the preceding claims, characterised in that the disubstituted butene is chosen from 1,4-diacetoxy-2-butene and 3,4-diacetoxy-1-butene and mixtures thereof.

**11.** Process according to any one of the preceding claims, characterised in that the palladium is introduced in the form of palladium chloride.